# EUROPEAN PATENT APPLICATION

(11) **EP 4 831 627 A1**
(43) Date of publication of application: **28.10.2026**
(21) Application number: 24906124.3
(22) Date of filing: 05.12.2024
(51) Int. Cl.: G01N 23/046

(54) **CT DEVICE AND DETECTION METHOD**

(30) Priority: 21.12.2023 CN 202311775565
(71) Applicant: Nuctech Company Limited, TongFang Building Shuangqinglu Haidian District Beijing 100084 (CN); Tsinghua University, Beijing 100084 (CN)
(72) Inventor: ZHANG, Li, Beijing 100084 (CN); CHEN, Zhiqiang, Beijing 100084 (CN); LI, Yuanjing, Beijing 100084 (CN); HUANG, Qingping, Beijing 100084 (CN); FENG, Bo, Beijing 100084 (CN); HE, Quan, Beijing 100084 (CN); GUO, Jin, Beijing 100084 (CN); XIE, Yunpeng, Beijing 100084 (CN)
(74) Representative: Cabinet Beau de Loménie
(86) International application number: PCT/CN2024/137107
(87) International publication number: WO 2025/130640

(57) **Abstract**

The present disclosure provides a CT device and a detection method. The CT device includes: a conveying apparatus; a rotating apparatus; a first control apparatus connected to the conveying apparatus, and configured to acquire a moving distance of the conveying apparatus and calculate, based on a moving speed of the conveying apparatus and a rotational speed of the rotating apparatus, a rotation angle of the rotating apparatus corresponding to the moving distance; a second control apparatus connected to the rotating apparatus, and configured to acquire a rotation angle of the rotating apparatus; and a calculation apparatus connected to the first control apparatus and the second control apparatus, and configured to receive the moving distance acquired by the first control apparatus, the rotation angle calculated by the first control apparatus, and the rotation angle acquired by the second control apparatus, associate the moving distance acquired by the first control apparatus with the rotation angle acquired by the second control apparatus in a one-to-one correspondence based on the rotation angle calculated by the first control apparatus to obtain synchronized data, and generate a radiographic image based on detection data and the synchronized data.

## Description

This application claims priority to Chinese Patent Application No. 202311775565.2, filed on December 21, 2023, the contents of which are incorporated herein by reference in their entirety.

### TECHNICAL FIELD

The present disclosure relates to a field of radiation detection technology, in particular to a CT device and a detection method for a CT device.

### BACKGROUND

Computed Tomography (CT) devices are widely used in fields such as medical treatment, security inspection, and industry.

During scanning operations, a CT device needs to acquire detection data from an X-ray machine, a rotation angle code of a detection apparatus, and a position code of an object being scanned, all corresponding to the same time instant, and generate a three-dimensional image based on the coded data from different times. In the related art, an angle controller for obtaining the angle code and a position controller for obtaining the position code generally communicate data via CAN communication, i.e., the position controller sends the position code to the angle controller, and the angle controller acquires the angle code and position code at the same time instant for the CT device to generate the three-dimensional image. This communication method has a certain latency, causing errors in the correspondence between the position code and the angle code. Furthermore, the position controller and the angle controller obtain the angle code and position code via carbon brushes. Carbon brushes are prone to wear and have a short service life, and thus require regular replacement, resulting in high maintenance costs.

### SUMMARY

The present disclosure provides a CT device and a detection method for a CT device.

According to an aspect of the present disclosure, a CT device is provided, including: a conveying apparatus configured to move a detection object in a first direction; a rotating apparatus disposed on a movement path of the conveying apparatus, and configured to rotate around the first direction at a preset speed, and emit X-rays towards the detection object and detect X-rays penetrating the detection object during rotation to generate detection data; a first control apparatus connected to the conveying apparatus, and configured to acquire a moving distance of the conveying apparatus, and calculate, based on a moving speed of the conveying apparatus and a rotational speed of the rotating apparatus, a rotation angle of the rotating apparatus corresponding to the moving distance; a second control apparatus connected to the rotating apparatus, and configured to acquire the rotation angle of the rotating apparatus; and a calculation apparatus connected to the first control apparatus and the second control apparatus, and configured to receive the moving distance acquired by the first control apparatus, the rotation angle calculated by the first control apparatus, and the rotation angle acquired by the second control apparatus, associate the moving distance acquired by the first control apparatus with the rotation angle acquired by the second control apparatus in a one-to-one correspondence based on the rotation angle calculated by the first control apparatus to obtain synchronized data, and generate a radiographic image based on the detection data and the synchronized data.

According to embodiments of the present disclosure, the CT device further includes: a first triggering apparatus mounted on the rotating apparatus and connected to the first control apparatus, and configured to provide a first trigger signal to the first control apparatus in response to the rotating apparatus rotating to a first predetermined position, where the first control apparatus is further configured to calculate a number of revolutions made by the rotating apparatus based on a number of times the first trigger signal is received, and calculate the rotation angle of the rotating apparatus corresponding to the moving distance based on the number of revolutions, the moving speed of the conveying apparatus, and the rotational speed of the rotating apparatus.

According to embodiments of the present disclosure, the first control apparatus is further configured to reset a previously calculated rotation angle of the rotating apparatus to zero in response to receiving the first trigger signal for a first time.

According to embodiments of the present disclosure, the CT device further includes: a second triggering apparatus mounted on the rotating apparatus and connected to the second control apparatus, and configured to provide a second trigger signal to the second control apparatus in response to the rotating apparatus rotating to a second predetermined position, where the second control apparatus is further configured to calculate a number of revolutions made by the rotating apparatus based on a number of times the second trigger signal is received, and calculate the rotation angle of the rotating apparatus based on the number of revolutions and a rotation angle of the rotating apparatus within a current revolution.

According to embodiments of the present disclosure, the second control apparatus is further configured to reset a previously acquired rotation angle to zero in response to receiving the second trigger signal for a first time.

According to embodiments of the present disclosure, the rotating apparatus includes a rotating portion and a fixed frame accommodating the rotating portion, the rotating portion being slidable relative to the fixed frame.

According to embodiments of the present disclosure, the first triggering apparatus includes: a first reference portion mounted at a first reference position on the rotating portion; and a first proximity switch mounted at a first detection position on the fixed frame and connected to the first control apparatus, the first proximity switch being configured to send the first trigger signal to the first control apparatus in response to a proximity between the first proximity switch and the first reference portion being less than a preset first threshold.

According to embodiments of the present disclosure, the second triggering apparatus includes: a second reference portion mounted at a second reference position on the fixed frame; and a second proximity switch mounted at a second detection position on the rotating portion and connected to the second control apparatus, the second proximity switch being configured to send the second trigger signal to the second control apparatus in response to a proximity between the second proximity switch and the second reference portion being less than a preset second threshold.

According to embodiments of the present disclosure, the first reference position and the second detection position are the same, and the first detection position and the second reference position are the same.

According to embodiments of the present disclosure, the CT device further includes: a photoelectric switch transmitter mounted on the fixed frame and connected to the first control apparatus; and a photoelectric switch receiver mounted on the rotating portion and connected to the second control apparatus, where the first control apparatus is further configured to, in response to receiving a synchronization instruction from the calculation apparatus, control the photoelectric switch transmitter to emit a photoelectric signal and reset a previously calculated number of revolutions of the rotating apparatus to zero upon receiving the first trigger signal; and where the second control apparatus is further configured to, in response to the photoelectric switch receiver receiving the photoelectric signal, reset a previously calculated number of revolutions of the rotating apparatus to zero upon receiving the second trigger signal.

According to embodiments of the present disclosure, calculating a unit rotation time as a time required for the rotating apparatus to rotate by a unit angle based on the rotational speed of the rotating apparatus; calculating a unit moving distance as a moving distance of the moving apparatus within the unit rotation time based on the moving speed of the conveying apparatus, and calculating a periodic moving distance as a moving distance of the moving apparatus within a time period for the rotating apparatus to complete one revolution; and calculating the rotation angle of the rotating apparatus based on the moving distance of the conveying apparatus according to: $Z = a + \left(n*D+m*Δd\right)$ $∅ = n * 360 + m * Δ φ$ where *Z* represents a code of the moving distance of the conveying apparatus, *Ø* represents a code of the rotation angle of the rotating apparatus, *n* represents a number of revolutions made by the rotating apparatus, *m* represents a factor by which the rotation angle of the rotating apparatus within a current revolution is a multiple of a unit rotation angle, *D* represents a number of pulses corresponding to the periodic moving distance of the conveying apparatus, Δ*d* represents a number of pulses corresponding to the unit moving distance of the conveying apparatus, Δ*φ* represents the unit rotation angle of the rotating apparatus, and *a* represents a number of pulses corresponding to a historical moving distance of the conveying apparatus.

According to embodiments of the present disclosure, the first control apparatus includes: a first sensor mounted on the conveying apparatus, and configured to acquire the moving distance of the conveying apparatus; and a first controller connected to the first sensor, and configured to receive the moving distance of the conveying apparatus acquired by the first sensor, and calculate the rotation angle of the rotating apparatus corresponding to the moving distance based on the moving speed of the conveying apparatus and the rotational speed of the rotating apparatus.

According to embodiments of the present disclosure, the second control apparatus includes: a second sensor mounted on the rotating apparatus, and configured to acquire the rotation angle of the rotating apparatus; and a second controller connected to the at least one second sensor, and configured to receive the rotation angle of the rotating apparatus acquired by the second sensor.

According to embodiments of the present disclosure, at least one of the first controller and the second controller is implemented by a Field Programmable Gate Array (FPGA).

According to embodiments of the present disclosure, the conveying apparatus includes a conveyor belt and a conveyor belt controller for controlling the conveyor belt to rotate, the first sensor includes a conveyor belt encoder, the conveyor belt encoder is connected to the conveyor belt controller, and the conveyor belt encoder is configured to rotate synchronously with the conveyor belt and emit pulses for calculating the moving distance of the conveyor belt to the first control apparatus during rotation.

According to embodiments of the present disclosure, the first control apparatus and the second control apparatus are configured to communicate data wirelessly.

According to another aspect of the present disclosure, a detection method for a CT device is provided, including: acquiring, by a first control apparatus, a moving distance of the conveying apparatus, and calculating, based on a moving speed of the conveying apparatus and a rotational speed of the rotating apparatus, a rotation angle of the rotating apparatus corresponding to the moving distance; acquiring, by a second control apparatus, the rotation angle of the rotating apparatus; and receiving, by a calculation apparatus, the moving distance acquired by the first control apparatus, the rotation angle calculated by the first control apparatus, and the rotation angle acquired by the second control apparatus, associating the moving distance acquired by the first control apparatus with the rotation angle acquired by the second control apparatus in one-to-one correspondence based on the rotation angle calculated by the first control apparatus to obtain synchronized data, and generating a radiographic image based on detection data and the synchronized data.

### BRIEF DESCRIPTION OF THE DRAWINGS

The above and other objectives, features and advantages of the present disclosure will be more apparent through the following description of embodiments of the present disclosure with reference to the accompanying drawings, wherein:
FIG. 1 schematically shows a structural block diagram of a CT device according to an embodiment of the present disclosure;
FIG. 2A schematically shows a structural block diagram of a rotating apparatus in a CT device according to an embodiment of the present disclosure;
FIG. 2B schematically shows another structural block diagram of a rotating apparatus in a CT device according to an embodiment of the present disclosure;
FIG. 2C schematically shows a structural block diagram of a photoelectric switch transmitter and a photoelectric switch receiver in a CT device according to an embodiment of the present disclosure;
FIG. 2D schematically shows a structural block diagram of a rotating apparatus in a CT device according to an embodiment of the present disclosure;
FIG. 3 schematically shows a structural block diagram of a rotating apparatus, a first control apparatus, and a second control apparatus in a CT device according to an embodiment of the present disclosure; and
FIG. 4 schematically shows a flowchart of a detection method for a CT device according to an embodiment of the present disclosure.

### DETAILED DESCRIPTION OF EMBODIMENTS

Embodiments of the present disclosure will be described below with reference to the accompanying drawings. It should be understood, however, that these descriptions are merely exemplary and are not intended to limit the scope of the present disclosure. In the following detailed descriptions, for purposes of explanation, numerous specific details are set forth in order to provide a thorough understanding of embodiments of the present disclosure. It is obvious, however, that one or more embodiments may be implemented without these specific details. In addition, in the following descriptions, descriptions of well-known structures and technologies are omitted to avoid unnecessarily obscuring the concept of the present disclosure.

Terms used herein are for the purpose of describing embodiments only and are not intended to limit the present disclosure. Terms "comprising", "including" and the like used herein specify a presence of the feature, step, operation and/or component, but do not preclude a presence or addition of one or more other features, steps, operations or components.

All terms (including technical and scientific terms) used herein have the meaning as commonly understood by those skilled in the art, unless otherwise defined. It should be noted that the terms used herein should be construed to have meanings consistent with the context of the present description and should not be construed in an idealized or overly rigid manner.

Where expressions like "at least one of A, B, and C, etc." are used, they should generally be interpreted in accordance with the meaning of the expression as commonly understood by those skilled in the art (e.g., "a system having at least one of A, B and C" should include, but not be limited to, a system having A alone, having B alone, having C alone, having A and B, having A and C, having B and C, and/or having A, B, C, etc.).

In the technical solutions of the present disclosure, the collection, storage, use, processing, transmission, provision, disclosure, application, and other processing of involved data (e.g., including but not limited to user personal information) comply with relevant laws and regulations, take necessary confidentiality measures, and do not violate public order or good customs.

Embodiments of the present disclosure provide a CT device and a detection method for a CT device.

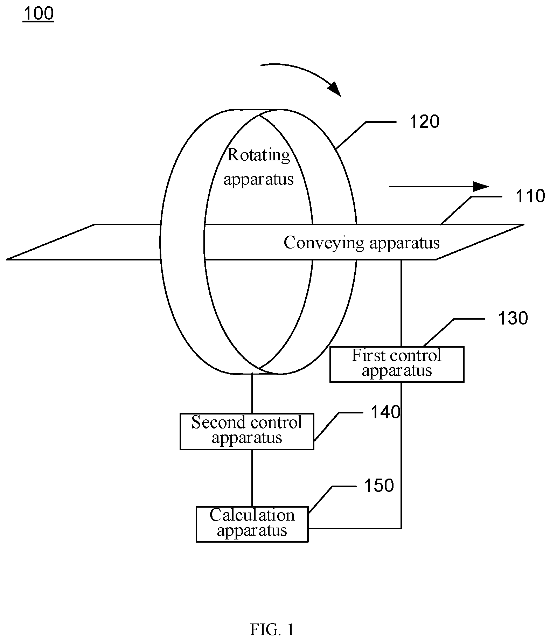

FIG. 1 schematically shows a structural block diagram of a CT device according to an embodiment of the present disclosure.

As shown in FIG. 1, a CT device 100 of the embodiment includes: a conveying apparatus 110, a rotating apparatus 120, a first control apparatus 130, a second control apparatus 140, and a calculation apparatus 150.

The conveying apparatus 110 is used to move a detection object in a first direction. The first direction refers to the direction along which the detection object placed on the conveying apparatus moves forward.

The rotating apparatus 120 is disposed on a movement path of the conveying apparatus and is used to rotate around the first direction at a preset speed, emit X-rays towards the detection object, and detect X-rays penetrating the detection object during rotation to generate detection data. When the conveying apparatus 110 moves the detection object in the first direction, the rotating apparatus 120 rotates around the conveying apparatus. The detection data includes a slice structure of the detection object at a position scanned by X-ray. As the detection object on the conveying apparatus continuously moves forward, slice structures of respective portions of the detection object are obtained as detection data.

The first control apparatus 130 is connected to the conveying apparatus and is used to acquire a moving distance of the conveying apparatus and calculate, based on a moving speed of the conveying apparatus and a rotational speed of the rotating apparatus, a rotation angle of the rotating apparatus corresponding to the moving distance. The moving distance may be represented by a code *Z*, and the rotation angle may be represented by a code *Ø*. Each time detection data is generated during rotation, a moving distance code and a rotation angle code corresponding to the detection data occur.

In some embodiments of the present disclosure, the first control apparatus may include a first sensor and a first controller. The first sensor is mounted on the conveying apparatus and is used to acquire the moving distance of the conveying apparatus. The first controller is connected to the first sensor and is used to receive the moving distance of the conveying apparatus acquired by the first sensor and calculate, based on the moving speed of the conveying apparatus and the rotational speed of the rotating apparatus, the rotation angle of the rotating apparatus corresponding to the moving distance. The first controller may be implemented by a Field Programmable Gate Array (FPGA).

In some embodiments of the present disclosure, the first sensor includes a conveyor belt encoder, and the conveying apparatus includes a conveyor belt and a conveyor belt controller for controlling the conveyor belt to rotate. The conveyor belt encoder is connected to the conveyor belt controller. The conveyor belt encoder rotates synchronously with the conveyor belt and emits pulses for calculating the moving distance of the conveyor belt to the first control apparatus during rotation.

The conveyor belt controller controls the conveyor belt to rotate, thereby driving the detection object on the conveyor belt to be displaced in the first direction. The conveyor belt encoder is used to represent the moving distance of the conveyor belt through pulse signals. Specifically, the conveyor belt encoder generates a periodic pulse signal during rotation, with a predetermined number of pulses being generated per revolution, so that the number of pulses may indicate the moving distance of the conveyor belt.

In some embodiments of the present disclosure, a time required for the rotating apparatus to rotate by a unit angle is calculated based on the rotational speed of the rotating apparatus, as a unit rotation time. A moving distance of the moving apparatus within the unit rotation time is calculated based on the moving speed of the conveying apparatus, as a unit moving distance. A moving distance of the moving apparatus within a time period for the rotating apparatus to complete one revolution is calculated, as a periodic moving distance. The rotation angle of the rotating apparatus is calculated based on the moving distance of the conveying apparatus according to the following formulas (1) and (2): $Z = a + \left(n*D+m*Δd\right)$ (1) $∅ = n * 360 + m * Δ φ$ (2) where *Z* represents a code of the moving distance of the conveying apparatus, *Ø* represents a code of the rotation angle of the rotating apparatus, *n* represents a number of revolutions made by the rotating apparatus, *m* represents a factor by which the rotation angle of the rotating apparatus within a current revolution is a multiple of a unit rotation angle, *D* represents a number of pulses corresponding to the periodic moving distance of the conveying apparatus, Δ*d* represents a number of pulses corresponding to the unit moving distance of the conveying apparatus, Δ*φ* represents the unit rotation angle of the rotating apparatus, and *a* represents a number of pulses corresponding to a historical moving distance of the conveying apparatus.

Assuming that the rotating apparatus rotates at a speed of 120 rpm, i.e. each revolution takes 500 milliseconds, and the unit rotation angle Δ*φ* is 36 degrees (1/10 revolution), the unit rotation time for the rotating apparatus to rotate by the unit rotation angle is 50 milliseconds. If the code *Z* of the moving distance of the moving apparatus corresponding to one revolution of the rotating apparatus is 1000, indicating that the moving apparatus emits 1000 pulses while moving in the first direction, then the code Δ*d* of the moving distance of the moving apparatus corresponding to the unit rotation time of the rotating apparatus is 100. The initial value *a* of the code of the moving distance is recorded as 10000 and the initial value of the code of the rotation angle is 0. After the rotating apparatus and the conveying apparatus start moving simultaneously, the moving distance of the conveying apparatus is recorded and rotation angle of the rotating apparatus is calculated when the rotating apparatus rotates by 1 unit rotation angle, 2 unit rotation angles, ..., *n* unit rotation angles.

As shown in the table below, when the rotating apparatus rotates by 1 unit rotation angle, the code of the rotation angle is 36, and the code of the corresponding moving distance is 10100. When the first control apparatus obtains the code *Z* of the moving distance of 10200 and receives 0 first trigger signals, the obtained number of revolutions *n* of the rotating apparatus is 0. According to formula (1), *m* = 2, indicating that the rotating apparatus has rotated by two unit rotation angles. Therefore, according to formula (2), the code *Ø* of the rotation angle with code *Z* of the moving distance being equal to 10200 is calculated as *Ø=* 0*360+2*36=72.

| Slip Ring Position | Code of Rotation Angle Calculated by First Control Apparatus | Code of Moving Distance Acquired by First Control Apparatus |
|---|---|---|
| 0/10-revolution position of the 0th revolution | 0*360+0*36=0 | 10000 |
| 1/10-revolution position of the 0th revolution | 0*360+1*36=36 | 10000+(0*1000+1*100)=10100 |
| ...... | | |
| m/10-revolution position of the nth revolution | n*360+m*36 | 10000+(n*1000+m*100) |

The second control apparatus 140 is connected to the rotating apparatus and is used to acquire the rotation angle of the rotating apparatus.

In some embodiments of the present disclosure, the second control apparatus 140 may include: a second sensor mounted on the rotating apparatus and used to acquire the rotation angle of the rotating apparatus; and a second controller connected to the at least one second sensor and used to receive the rotation angle of the rotating apparatus acquired by the second sensor. In some embodiments, the second control apparatus 140 may further receive the detection data generated by the rotating apparatus and provide the detection data to the calculation apparatus 150 for use by the calculation apparatus 150 in imaging. In some embodiments, the detection data generated by the rotating apparatus may be acquired by a separate acquisition apparatus (e.g., a data acquisition card) and provided to the calculation apparatus 150.

The second sensor may be an angle sensor. The second control apparatus controls the second sensor to acquire the code *Ø* of the rotation angle of the rotating apparatus. The code *Ø* of the rotation angle may be initialized to 0 by the second controller. The second controller may be implemented by a Field Programmable Gate Array (FPGA).

In some embodiments of the present disclosure, the first control apparatus 130 and the second control apparatus 140 may communicate data wirelessly. The data may include the moving distance of the moving apparatus, the rotation angle of the rotating apparatus, the number of revolutions made by the rotating apparatus, etc., which are used for associating the codes of the moving distance with the codes of the rotation angle in one-to-one correspondence, so as to achieve synchronization between the code of the moving distance and the code of the rotation angle.

The calculation apparatus 150 is connected to the first control apparatus and the second control apparatus and is used to receive the moving distance acquired by the first control apparatus, the rotation angle calculated by the first control apparatus, and the rotation angle acquired by the second control apparatus, associate the moving distance acquired by the first control apparatus with the rotation angle acquired by the second control apparatus in a one-to-one correspondence based on the rotation angle calculated by the first control apparatus to obtain synchronized data, and generate a radiographic image based on detection data and the synchronized data. The calculation apparatus 150 may be a host computer and may be implemented by a general-purpose computer.

The moving distances acquired by the first control apparatus and the rotation angles acquired by the second control apparatus are both sent to the calculation apparatus in real time. The first control apparatus may calculate the rotation angle based on the moving distance at fixed time intervals. For the current acquired moving distance, the first control apparatus sends the moving distance and the rotation angle calculated from the moving distance to the calculation apparatus. The calculation apparatus associates the moving distance with the rotation angle, which is acquired by the second control apparatus and matches the rotation angle sent by the first control apparatus, so as to obtain the moving distance synchronized with rotation angle for the current detection data. Each piece of detection data includes slice data of the detection object at a position. Based on all detection data and the corresponding moving distances and rotation angles, a complete radiographic image of the detection object is generated.

In embodiments of the present disclosure, the first control apparatus not only acquires the moving distance of the conveying apparatus but also calculates, based on the moving speed of the conveying apparatus and the rotational speed of the rotating apparatus, the rotation angle of the rotating apparatus corresponding to the moving distance. The moving distance of the moving apparatus may be synchronized with the rotation angle of the rotating apparatus, without communication between the first control apparatus and the second control apparatus or through wireless communication between the two control apparatuses. This improves the real-time performance and accuracy of data transmission as compared to traditional CAN communication. Additionally, since the carbon brushes used in CAN communication are eliminated, equipment wear and maintenance costs are reduced.

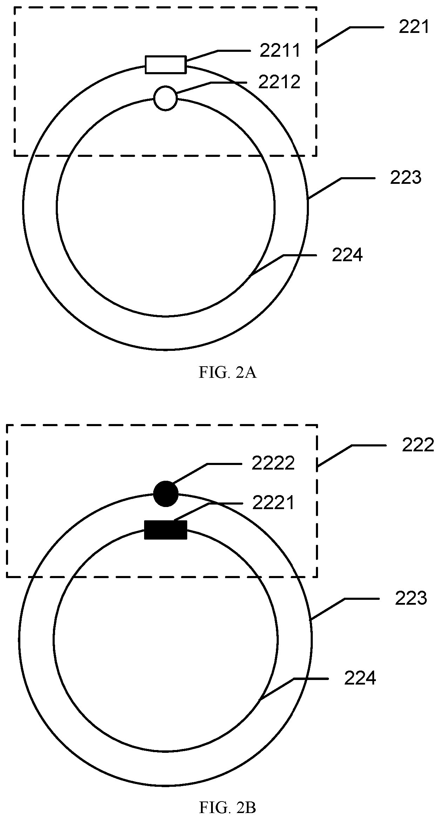

FIG. 2A schematically shows a structural block diagram of a rotating apparatus in a CT device according to an embodiment of the present disclosure.

As shown in FIG. 2A, the rotating apparatus 120 may include a rotating portion 224 (also referred to as a slip ring) and a fixed frame 223 accommodating the rotating portion. The rotating portion 224 is slidable relative to the fixed frame 223. The rotating portion 224 may be provided with an X-ray emitter and an X-ray detector. The X-ray emitter emits X-rays during the rotation of the rotating portion 224, and the X-ray detector receives the X-rays. Since X-rays are attenuated differently after penetrating objects of different materials, the X-ray detector generates different detection data based on such attenuation for later imaging.

The rotating apparatus 120 further includes a first triggering apparatus 221 used to provide a first trigger signal to the first control apparatus when the rotating apparatus rotates to a first predetermined position. For example, as shown in FIG. 2A, the first triggering apparatus 221 may include a first proximity switch 2211 and a first reference portion 2212. The first reference portion 2212 is mounted at a first reference position on the rotating portion 224 of the rotating apparatus 120. The first proximity switch 2211 is mounted at a first detection position on the fixed frame 223 of the rotating apparatus 120. The first trigger signal is a signal sent when the proximity between the first proximity switch and the first reference portion is less than a preset first threshold. The first trigger signal indicates that the first reference position on the rotating portion aligns with the first detection position on the fixed frame during the rotation of the rotating portion. Therefore, the number of times the first control apparatus receives the first trigger signal may indicate the number of revolutions made by the rotating apparatus.

The first control apparatus may calculate the number of revolutions made by the rotating apparatus based on the number of times the first trigger signal is received, and calculate the rotation angle of the rotating apparatus corresponding to the moving distance based on the number of revolutions, the moving speed of the conveying apparatus, and the rotational speed of the rotating apparatus. In some embodiments of the present disclosure, the first control apparatus may further reset a previously calculated rotation angle of the rotating apparatus to zero upon receiving the first trigger signal for a first time.

For example, after the rotating apparatus starts rotating, the first proximity switch mounted at the first reference position on the rotating portion starts rotating clockwise together with the rotating portion. When the first proximity switch passes the first reference portion mounted at the first detection position on the fixed frame for the first time, the first proximity switch sends a first trigger signal to the first control apparatus. The first control apparatus resets the previously calculated rotation angle to zero. Subsequently, the first proximity switch rotates with the rotating portion at a speed of 120 rpm for 10 seconds and sends the first trigger signal to the first control apparatus 20 times, indicating that the rotating apparatus has completed 20 revolutions. At this time, the first control apparatus calculates the code of the rotation angle according to formula (1) with *n* =20.

FIG. 2B schematically shows another structural block diagram of a rotating apparatus in a CT device according to an embodiment of the present disclosure.

As shown in FIG. 2B, in addition to the rotating portion 224 and the fixed frame 223 accommodating the rotating portion, the rotating apparatus 120 may include a second triggering apparatus 222. The second triggering apparatus 222 is used to provide a second trigger signal to the second control apparatus when the rotating apparatus rotates to a second predetermined position. In some embodiments, the second control apparatus 222 may further be used to calculate the number of revolutions made by the rotating apparatus based on the number of times the second trigger signal is received, and calculate the rotation angle of the rotating apparatus based on the number of revolutions and a rotation angle of the rotating apparatus within a current revolution.

The second triggering apparatus 222 may include a second proximity switch 2221 and a second reference portion 2222. The second reference portion 2222 is mounted at a second reference position on the fixed frame 223 of the rotating apparatus 120. The second proximity switch 2221 is mounted at a second detection position on the rotating portion 224 of the rotating apparatus 120. The second trigger signal is a signal sent when the proximity between the second proximity switch and the second reference portion is less than a preset second threshold, indicating that the second detection position on the rotating portion aligns with the second reference position on the fixed frame during the rotation of the rotating portion. Therefore, the number of times the second control apparatus receives the second trigger signal indicates the number of revolutions made by the rotating apparatus. In some embodiments of the present disclosure, the second control apparatus is further used to reset a previously acquired rotation angle to zero upon receiving the second trigger signal for a first time.

For example, after the rotating apparatus starts rotating, the second reference portion mounted at the second detection position on the rotating portion starts rotating clockwise with the rotating portion. When the second proximity switch mounted at the second reference position on the fixed frame aligns with the second reference portion for the first time, the second proximity switch sends a second trigger signal to the second control apparatus, and the second control apparatus resets the previously calculated rotation angle to zero. Subsequently, the second reference portion rotates with the rotating portion at a speed of 120 rpm for 10 seconds. The second proximity switch detects 20 approaches of the second reference portion and thus sends the second trigger signal to the second control apparatus 20 times. The second control apparatus calculates the code of the rotation angle based on the number of revolutions and the rotation time.

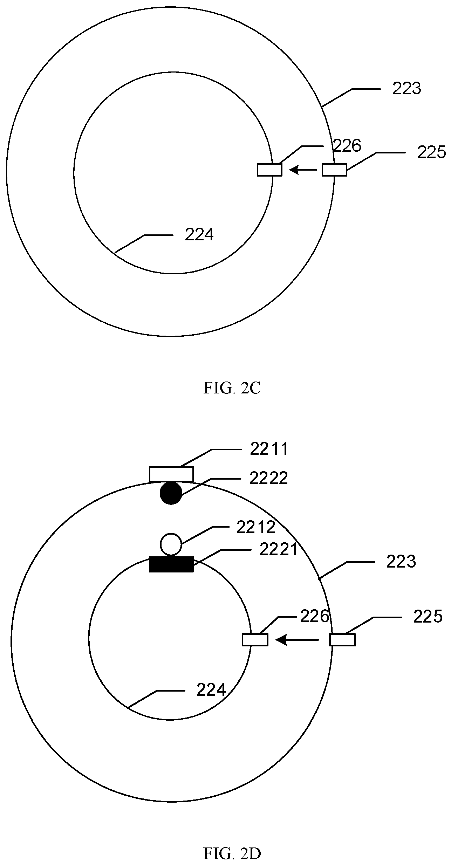

FIG. 2C schematically shows a structural block diagram of a photoelectric switch transmitter and a photoelectric switch receiver in a CT device according to an embodiment of the present disclosure.

As shown in FIG. 2C, in addition to including the rotating portion 224 and the fixed frame 223, the rotating apparatus 120 may include a photoelectric switch transmitter 225 and a photoelectric switch receiver 226.

The photoelectric switch transmitter 225 is mounted on the fixed frame 223 and connected to the first control apparatus 130. The photoelectric switch receiver 226 is mounted on the rotating portion 224 and connected to the second control apparatus 140. The first control apparatus 130 may, in response to receiving a synchronization instruction from the calculation apparatus 150, control the photoelectric switch transmitter 225 to emit a photoelectric signal and reset a previously calculated rotation angle of the rotating apparatus to zero upon receiving the first trigger signal. The second control apparatus 140 may, in response to the photoelectric switch receiver 226 receiving the photoelectric signal, reset a previously calculated rotation angle of the rotating apparatus to zero upon receiving the second trigger signal.

For example, when a new detection object is placed on the conveying apparatus and the CT device is about to start detection, the first control apparatus 130 receives the synchronization instruction from the calculation apparatus 150, turns on the photoelectric switch transmitter 225 to emit a photoelectric signal, and waits for the first trigger signal. The first control apparatus 130 resets the previously calculated rotation angle of the rotating apparatus to zero upon receiving the first trigger signal. After the photoelectric switch receiver 226 receives the photoelectric signal, the second control apparatus 140 resets the previously calculated rotation angle of the rotating apparatus to zero upon receiving the second trigger signal. After synchronizing the codes of the first control apparatus and the second control apparatus, as the conveying apparatus moves the detection object in the first direction, the rotating apparatus rotates around the conveying apparatus and emits and receives X-rays to generate detection data of the detection object. The first control apparatus and the second control apparatus determine the moving distance and rotation angle corresponding to the detection data. The calculation apparatus obtains synchronized data based on the moving distance and the rotation angle, and generates a radiographic image of the detection object based on the detection data and the synchronized data. Thus, the calculated rotation angles of the first control apparatus and the second control apparatus may be reset to zero, and data synchronization during subsequent detection may be achieved.

FIG. 2D schematically shows a structural block diagram of a rotating apparatus in a CT device according to an embodiment of the present disclosure.

As shown in FIG. 2D, the rotating apparatus 120 includes a rotating portion 224, a fixed frame 223, a first triggering apparatus, a second triggering apparatus, a photoelectric switch transmitter 225, and a photoelectric switch receiver 226. The first triggering apparatus includes a first proximity switch 2211 and a first reference portion. The second triggering apparatus includes a second proximity switch 2221 and a second reference portion 2222. In some embodiments, the descriptions of the rotating portion, the fixed frame, the first triggering apparatus, the second triggering apparatus, the photoelectric switch transmitter, and the photoelectric switch receiver above also apply to the embodiment.

As shown in FIG. 2D, the first reference position where the first reference portion 2212 of the first triggering apparatus is located is the same as the second detection position where the second proximity switch 2221 of the second triggering apparatus is located. The first detection position where the first proximity switch 2211 of the first triggering apparatus is located is the same as the second reference position where the second reference portion 2222 of the second triggering apparatus is located. Therefore, after the photoelectric switch transmitter emits the photoelectric signal, the first triggering apparatus and the second triggering apparatus are triggered at the same time due to their positional relationship and respectively send the first trigger signal and the second trigger signal, simultaneously resetting the rotation angles of the rotating apparatus previously calculated by the first control apparatus and the second control apparatus to zero, thereby achieving data synchronization.

The first triggering apparatus and the second triggering apparatus are respectively connected to the first control apparatus and the second control apparatus, ensuring that the two triggering apparatuses send the first trigger signal and the second trigger signal at the same time, thereby keeping the synchronization between the number of revolutions and codes of the rotation angle calculated by the first control apparatus and the second control apparatus.

The photoelectric switch transmitter 225 and the photoelectric switch receiver 226 may be installed at positions as desired, for example, positions different from the first triggering apparatus and the second triggering apparatus. In some embodiments, the photoelectric switch transmitter 225 and the photoelectric switch receiver 226 may be disposed upstream of the first triggering apparatus and the second triggering apparatus along the rotation direction of the rotating portion, and may be spaced apart from the first triggering apparatus and the second triggering apparatus by a predetermined distance. Accordingly, the photoelectric switch transmitter 225 and the photoelectric switch receiver 226 inform the first control apparatus that a new round of detection is about to start, and then the first triggering apparatus and the second triggering apparatus are used to achieve the first trigger and the resetting of historical data.

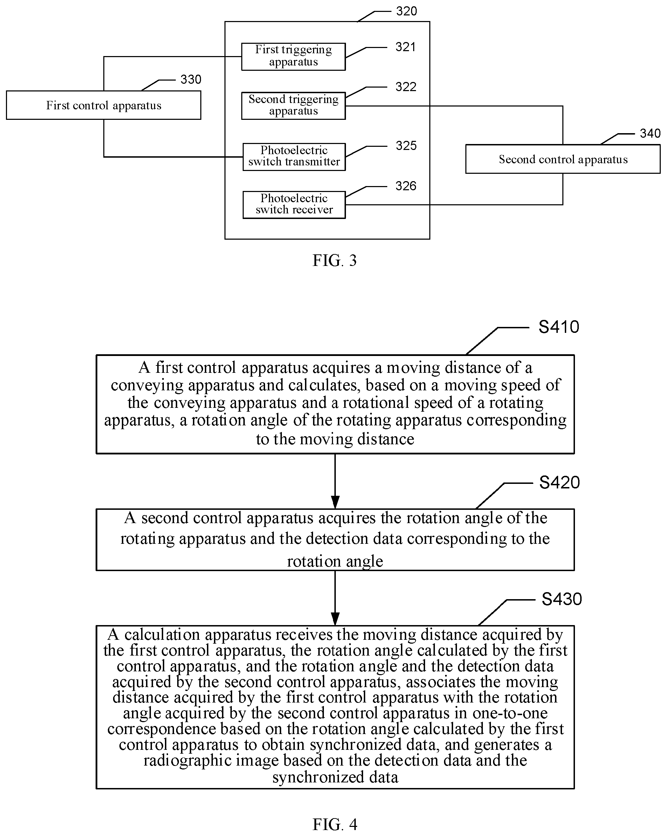

FIG. 3 schematically shows a structural block diagram of a rotating apparatus, a first control apparatus, and a second control apparatus in a CT device according to an embodiment of the present disclosure.

As shown in FIG. 3, in some embodiments of the present disclosure, the rotating apparatus 320 includes a first triggering apparatus 321, a second triggering apparatus 322, a photoelectric switch transmitter 325, and a photoelectric switch receiver 326. The first triggering apparatus 321 and the photoelectric switch transmitter 325 are both connected to the first control apparatus 330. The second triggering apparatus 322 and the photoelectric switch receiver 326 are both connected to the second control apparatus 340.

In some embodiments of the present disclosure, the first triggering apparatus 321 includes a first proximity switch and a first reference portion. The first reference portion is mounted at a first reference position on the rotating portion. The first proximity switch is mounted at a first detection position on the fixed frame and connected to the first control apparatus 330. The second triggering apparatus 322 includes a second proximity switch and a second reference portion. The second reference portion is mounted at a second reference position on the fixed frame. The second proximity switch is mounted at a second detection position on the rotating portion and connected to the second control apparatus 340.

FIG. 4 schematically shows a flowchart of a detection method for a CT device according to an embodiment of the present disclosure.

In step S410, a first control apparatus acquires a moving distance of a conveying apparatus and calculates, based on a moving speed of the conveying apparatus and a rotational speed of a rotating apparatus, a rotation angle of the rotating apparatus corresponding to the moving distance.

In step S420, a second control apparatus acquires the rotation angle of the rotating apparatus.

In step S430, a calculation apparatus receives the moving distance acquired by the first control apparatus, the rotation angle calculated by the first control apparatus, and the rotation angle acquired by the second control apparatus, associates the moving distance acquired by the first control apparatus with the rotation angle acquired by the second control apparatus in a one-to-one correspondence based on the rotation angle calculated by the first control apparatus to obtain synchronized data, and generates a radiographic image based on detection data and the synchronized data.

It should also be noted that directional terms mentioned in the embodiments, such as "up", "down", "front", "back", "left", "right", etc., are only the directions referring to the accompanying drawings and are not intended to limit the scope of protection of the present disclosure. Throughout the accompanying drawings, the same elements are represented by the same or similar reference signs. Conventional structures or constructions will be omitted when they may obscure the understanding of the present disclosure.

In addition, shapes and sizes of the respective components in the figures do not reflect true sizes and proportions, but merely illustrate contents of embodiments of the present disclosure. Moreover, in the claims, any reference signs placed between parentheses should not be construed as limiting the claims.

Furthermore, the word "including" does not exclude the presence of elements or steps not listed in the claims. The word "a" or "an" preceding an element does not exclude the presence of a plurality of such elements.

The use of ordinal numbers such as "first", "second", "third", etc. in the specification and claims to modify a corresponding element does not mean that the element has any ordinal number, nor does it represent the ordering of one element relative to another element or the ordering of manufacturing methods. The use of such ordinal numbers is only used to clearly distinguish an element having a certain name from another element having the same name.

Those skilled in the art will appreciate that features recited in the various embodiments of the present disclosure and/or the claims may be combined and/or incorporated in a variety of ways, even if such combinations or incorporations are not clearly recited in the present disclosure. In particular, the features recited in the various embodiments of the present disclosure and/or the claims may be combined and/or incorporated in a variety of ways without departing from the spirit and teachings of the present disclosure, and all such combinations and/or incorporations fall within the scope of the present disclosure.

Embodiments of the present disclosure have been described above. However, these embodiments are for illustrative purposes only, and are not intended to limit the scope of the present disclosure. Although the various embodiments are described above separately, this does not mean that the measures in the various embodiments may not be advantageously used in combination. The scope of the present disclosure is defined by the appended claims and their equivalents. Without departing from the scope of the present disclosure, those skilled in the art may make various substitutions and modifications, and these substitutions and modifications should all fall within the scope of the present disclosure.

## Claims

1. A CT device, comprising:
a conveying apparatus configured to move a detection object in a first direction;
a rotating apparatus disposed on a movement path of the conveying apparatus, and configured to rotate around the first direction at a preset speed, and emit X-rays towards the detection object and detect X-rays penetrating the detection object during rotation to generate detection data;
a first control apparatus connected to the conveying apparatus, and configured to acquire a moving distance of the conveying apparatus, and calculate, based on a moving speed of the conveying apparatus and a rotational speed of the rotating apparatus, a rotation angle of the rotating apparatus corresponding to the moving distance;
a second control apparatus connected to the rotating apparatus, and configured to acquire a rotation angle of the rotating apparatus; and
a calculation apparatus connected to the first control apparatus and the second control apparatus, and configured to receive the moving distance acquired by the first control apparatus, the rotation angle calculated by the first control apparatus, and the rotation angle acquired by the second control apparatus, associate the moving distance acquired by the first control apparatus with the rotation angle acquired by the second control apparatus in a one-to-one correspondence based on the rotation angle calculated by the first control apparatus to obtain synchronized data, and generate a radiographic image based on the detection data and the synchronized data.

2. The CT device according to claim 1, further comprising: a first triggering apparatus mounted on the rotating apparatus and connected to the first control apparatus, and configured to provide a first trigger signal to the first control apparatus in response to the rotating apparatus rotating to a first predetermined position,
wherein the first control apparatus is further configured to calculate a number of revolutions made by the rotating apparatus based on a number of times the first trigger signal is received, and calculate the rotation angle of the rotating apparatus corresponding to the moving distance based on the number of revolutions, the moving speed of the conveying apparatus, and the rotational speed of the rotating apparatus.

3. The CT device according to claim 2, wherein the first control apparatus is further configured to reset a previously calculated rotation angle of the rotating apparatus to zero in response to receiving the first trigger signal for a first time.

4. The CT device according to any one of claims 1 to 3, further comprising: a second triggering apparatus mounted on the rotating apparatus and connected to the second control apparatus, and configured to provide a second trigger signal to the second control apparatus in response to the rotating apparatus rotating to a second predetermined position,
wherein the second control apparatus is further configured to calculate a number of revolutions made by the rotating apparatus based on a number of times the second trigger signal is received, and calculate the rotation angle of the rotating apparatus based on the number of revolutions and a rotation angle of the rotating apparatus within a current revolution.

5. The CT device according to claim 4, wherein the second control apparatus is further configured to reset a previously acquired rotation angle to zero in response to receiving the second trigger signal for a first time.

6. The CT device according to any one of claims 1 to 5, wherein the rotating apparatus comprises a rotating portion and a fixed frame accommodating the rotating portion, the rotating portion being slidable relative to the fixed frame.

7. The CT device according to claim 6, wherein the first triggering apparatus comprises:
a first reference portion mounted at a first reference position on the rotating portion; and
a first proximity switch mounted at a first detection position on the fixed frame and connected to the first control apparatus, the first proximity switch being configured to send the first trigger signal to the first control apparatus in response to a proximity between the first proximity switch and the first reference portion being less than a preset first threshold.

8. The CT device according to claim 6 or 7, wherein the second triggering apparatus comprises:
a second reference portion mounted at a second reference position on the fixed frame; and
a second proximity switch mounted at a second detection position on the rotating portion and connected to the second control apparatus, the second proximity switch being configured to send the second trigger signal to the second control apparatus in response to a proximity between the second proximity switch and the second reference portion being less than a preset second threshold.

9. The CT device according to claim 8, wherein the first reference position and the second detection position are the same, and the first detection position and the second reference position are the same.

10. The CT device according to any one of claims 2 to 9, further comprising:
a photoelectric switch transmitter mounted on the fixed frame and connected to the first control apparatus; and
a photoelectric switch receiver mounted on the rotating portion and connected to the second control apparatus,
wherein the first control apparatus is further configured to, in response to receiving a synchronization instruction from the calculation apparatus, control the photoelectric switch transmitter to emit a photoelectric signal and reset a previously calculated rotation angle of the rotating apparatus to zero upon receiving the first trigger signal; and
wherein the second control apparatus is further configured to, in response to the photoelectric switch receiver receiving the photoelectric signal, reset a previously calculated rotation angle of the rotating apparatus to zero upon receiving the second trigger signal.

11. The CT device according to any one of claims 1 to 10, wherein the first control apparatus is configured to calculate, based on the moving speed of the conveying apparatus and the rotational speed of the rotating apparatus, the rotation angle of the rotating apparatus corresponding to the moving distance by:
calculating a unit rotation time as a time required for the rotating apparatus to rotate by a unit angle based on the rotational speed of the rotating apparatus;
calculating a unit moving distance as a moving distance of the moving apparatus within the unit rotation time based on the moving speed of the conveying apparatus, and calculating a periodic moving distance as a moving distance of the moving apparatus within a time period for the rotating apparatus to complete one revolution; and
calculating the rotation angle of the rotating apparatus based on the moving distance of the conveying apparatus according to: $Z = a + \left(n*D+m*Δd\right)$ $∅ = n * 360 + m * Δ φ$
wherein *Z* represents a code of the moving distance of the conveying apparatus, *Ø* represents a code of the rotation angle of the rotating apparatus, *n* represents a number of revolutions made by the rotating apparatus, *m* represents a factor by which the rotation angle of the rotating apparatus within a current revolution is a multiple of a unit rotation angle, *D* represents a number of pulses corresponding to the periodic moving distance of the conveying apparatus, Δ*d* represents a number of pulses corresponding to the unit moving distance of the conveying apparatus, Δ*φ* represents the unit rotation angle of the rotating apparatus, and *a* represents a number of pulses corresponding to a historical moving distance of the conveying apparatus.

12. The CT device according to any one of claims 1 to 11, wherein the first control apparatus comprises:
a first sensor mounted on the conveying apparatus, and configured to acquire the moving distance of the conveying apparatus; and
a first controller connected to the first sensor, and configured to receive the moving distance of the conveying apparatus acquired by the first sensor, and calculate the rotation angle of the rotating apparatus corresponding to the moving distance based on the moving speed of the conveying apparatus and the rotational speed of the rotating apparatus.

13. The CT device according to any one of claims 1 to 12, wherein the second control apparatus comprises:
a second sensor mounted on the rotating apparatus, and configured to acquire the rotation angle of the rotating apparatus; and
a second controller connected to the at least one second sensor, and configured to receive the rotation angle of the rotating apparatus acquired by the second sensor.

14. The CT device according to claim 12 or 13, wherein at least one of the first controller and the second controller is implemented by a Field Programmable Gate Array (FPGA).

15. The CT device according to any one of claims 1 to 14, wherein the conveying apparatus comprises a conveyor belt and a conveyor belt controller for controlling the conveyor belt to rotate, the first sensor comprises a conveyor belt encoder, the conveyor belt encoder is connected to the conveyor belt controller, and the conveyor belt encoder is configured to rotate synchronously with the conveyor belt and emit pulses for calculating the moving distance of the conveyor belt to the first control apparatus during rotation.

16. The CT device according to any one of claims 1 to 15, wherein the first control apparatus and the second control apparatus are configured to communicate data wirelessly.

17. A detection method for a CT device according to any one of claims 1 to 16, comprising:
acquiring, by a first control apparatus, a moving distance of the conveying apparatus, and calculating, based on a moving speed of the conveying apparatus and a rotational speed of the rotating apparatus, a rotation angle of the rotating apparatus corresponding to the moving distance;
acquiring, by a second control apparatus, a rotation angle of the rotating apparatus; and
receiving, by a calculation apparatus, the moving distance acquired by the first control apparatus, the rotation angle calculated by the first control apparatus, and the rotation angle acquired by the second control apparatus, associating the moving distance acquired by the first control apparatus with the rotation angle acquired by the second control apparatus in one-to-one correspondence based on the rotation angle calculated by the first control apparatus to obtain synchronized data, and generating a radiographic image based on detection data and the synchronized data.
